# EUROPEAN PATENT SPECIFICATION

(11) **EP 0 512 951 B1**
(45) Date of publication and mention of the grant of the patent: **23.07.1997**
(21) Application number: 92810304.3
(22) Date of filing: 28.04.1992
(51) Int. Cl.: C07D 401/12, C07D 211/94, C08K 5/3435, C08K 5/00

(54) **Substituted 1-oxy-4-acyloxypiperidine and 1-oxy-4-acylaminopiperidine stabilizers**
Substituierte 1-oxy-4-acyloxypiperidin und 1-oxy-4-acylaminopiperidin Stabilisatoren
1-oxy-4-acyloxypipéridines et 1-oxy-4-acylaminopipéridines substituées utilisables comme stabilisants

(30) Priority: 07.05.1991 US 697134
(43) Date of publication of application: 11.11.1992
(73) Proprietor: Ciba Specialty Chemicals Holding Inc., 4057 Basel (CH)
(72) Inventor: Odorisio, Paul A., Edgewater, NJ 07020 (US); Shum, Sai P., Pleasantville, NY 10572 (US)

(56) References cited:
- EP-A- 0 138 767
- EP-A- 0 389 424
- EP-A- 0 427 672
- Degradation and stabilisation of polyolefins,1983,p. 299
- Plastics additives handbook,1990,3rd edition, p. 195

## Description

The present invention pertains to novel 1-oxy-4-acyloxypiperidines and 1-oxy-4-acylaminopiperidines and their use as stabilizers for various polymeric substrates during processing to provide superior melt flow stabilization.

### Background of the Invention

The substituted 1-oxy-4-acyloxypiperidines and 1-oxy-4-acylaminopiperidines are novel compounds as no examples of said compounds have been reported in the patent or the chemical literature.

1-Hydroxy(alkoxy)-2,2,6,6-tetramethyl-4-acyloxypiperidines and other 1-hydroxy(alkoxy)-2,2,6,6-tetramethylpiperidine derivatives are known as effective light stabilizers, but said compounds are not known to be particularly effective as processing stabilizers. Said compounds are also structurally distinguished from the instant compounds of this invention.

1-Hydroxy-2,6-dialkylpiperidines are disclosed to prevent discoloration of phenolic antioxidants in United States Patent No. 4,316,996.

Substituted hydroxylamines, specifically N-hydroxypiperidine, are disclosed as effective antioxidants in United States Patent No. 3,644,278.

Other structurally distinct hydroxylamines have been disclosed as processing stabilizers as seen in United States Patent Nos. 4,668,721; 4,782,105; 4,876,300 and 4,898,901.

The instant compounds are structurally distinguished from each of the compounds disclosed in these prior art references and exhibit surprising superior process stabilization properties for polymeric substrates.

### Objects of the Invention

One object of the instant invention is to provide new substituted 1-oxy-4-acyloxypiperidines and 1-oxy-4-acylaminopiperidines which have superior process stabilizing properties for polymeric substrates.

Another object of the invention is to provide stabilized compositions containing the substituted 1-oxy-4-acyloxypiperidines and 1-oxy-4-acylaminopiperidines of this invention.

### Detailed Disclosure

The instant invention pertains to novel substituted 1-oxy-4-acyloxypiperidines or 1-oxy-4-acylaminopiperidines of formula I 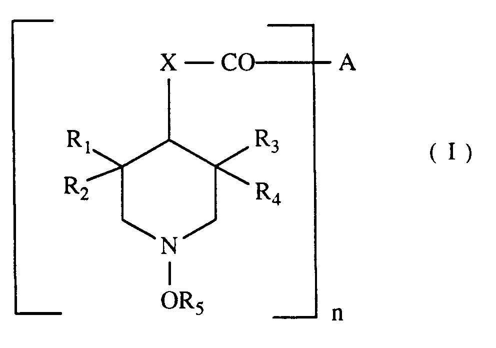 wherein
R₁, R₂, R₃ and R₄ are independently hydrogen; a linear or branched alkyl of 1 to 30 carbon atoms; said alkyl optionally terminated with -OR₆, -NR₇R₈, -SR₉, -COOR₁₀ or -CONR₁₁R₁₂, where R₆, R₇, R₈, R₉ and R₁₀ are independently alkyl of 1 to 20 carbon atoms or alkenyl of 3 to 18 carbon atons, and R₁₁ and R₁₂ are independently hydrogen or the same meaning as R₆; or said alkyl interrupted by one or more -O-, -S-, -SO-, -SO₂-, -CO-, -COO-, -OCO-, -CONR₁₃-, -NR₁₃CO- or -NR₁₄- where R₁₃ and R₁₄ have the same meaning as R₁₁; alkenyl of 3 to 20 carbon atoms; aryl of 6 to 10 carbon atoms; or said aryl substituted by one to three substituents selected from the group consisting of alkyl of 1 to 20 carbon atoms, cycloalkyl of 5 to 12 carbon atoms and phenylalkyl of 7 to 15 carbon atoms;
R₅ is hydrogen, alkyl of 1 to 18 carbon atoms, cycloalkyl of 5 to 12 carbon atoms, allyl or benzyl;
X is -O- or -NE-,
E is hydrogen, alkyl of 1 to 18 carbon atoms or cycloalkyl of 5 to 12 carbon atoms,
A is a direct bond; an n-valent linear or branched aliphatic hydrocarbon radical containing 1 to 20 carbon atoms, or said radical interrupted by one or more -O-, -S-, -SO-, -SO₂-, -CO-, -COO-, -OCO-, -CONR₁₅-, -NR₁₅CO- or -NR₁₆- where R₁₅ and R₁₆ have the same meaning as R₁₁; an n-valent aromatic or aromatic- aliphatic hydrocarbon of 6 to 30 carbon atoms, or said radical interrupted by one or more -O-, -S-, -SO-,-SO₂-, -CO-, -COO-, -OCO-, -CONR₁₇, -NR₁₇CO- or -NR₁₈- where R₁₇ and R₁₈ have the same meaning as R₁₁; or A can be -O-, -S-, -NR₁₉- where R₁₉ has the same meaning as R₁₁; and
n is an integer of 1 to 4.

All possible geometric isomers and stereoisomers which are predictable are to be included in the scope of this invention.

Preferably R₁, R₂, R₃ and R₄ are independently hydrogen or methyl; most preferably hydrogen.

Preferably R₅ is hydrogen, allyl or benzyl.

Preferably n is 1 or 2.

Preferably X is -O-.

Preferably A is alkyl of 1 to 18 carbon atoms when n is 1; and alkylene of 2 to 12 carbon atoms when n is 2.

Most preferably A is alkyl of 11 to 17 carbon atoms when n is 1; and alkylene of 2 to 8 carbon atoms when n is 2.

Most preferably R₅ is hydrogen or allyl.

Also preferred are compounds of formula I wherein R₁, R₂, R₃ and R₄ are independently hydrogen or methyl, n is 1 or 2, A is alkyl of 11 to 18 carbon atoms when n is 1, and alkylene of 2 to 12 carbon atoms when n is 2, and R₅ is hydrogen, allyl or benzyl.

The instant invention also pertains to stabilized compositions which comprise
(a) a synthetic polymer subject to thermal or oxidative degradation during processing, and
(b) an effective stabilizing amount of a compound of formula I as defined above.

The invention therefore also pertains to a method for stabilizing a synthetic polymer against damage by thermal or oxidative degradation during processing, wherein a compound of formula I is added thereto as stabilizer, and to the use of said compound as stabilizer, especially as process stabilizer for synthetic polymers.

Compositions wherein the synthetic polymer is a polyolefin are preferred.

The compounds of the instant invention are conveniently prepared by the reaction of a 4-hydroxypiperidine with an allyl halide, benzyl halide or lower alkyl acrylate followed by oxidation of the product formed with a peroxy compound, such as meta-chloroperbenzoic acid, to the corresponding 1-oxy compound. This is followed by a classic acylation of the 4-hydroxy group with an acid halide or acid anhydride.

The starting materials and intermediates used to make the instant compounds are largely items of commerce or can be made by methods known in the art.

The starting 4-hydroxypiperidines are obtained by reduction of the corresponding piperid-4-ones which in turn are readily accessible by classical synthetic methods as seen in A. R. Katritsky and C. W. Rees, Comprehensive Heterocyclic Chemistry, Vol. 2, pp 74, 95-96, 417-418 and 481 (1984); and Sir Derek Barton and W. D. Ollis, Comprehensive Organic Chemistry, Vol. 4, pp 42-43 and 64-65 (1979).

When any of R₁ to R₁₉, E or A is alkyl, such alkyl groups are, for example, methyl, ethyl, propyl, isopropyl, n-butyl, isobutyl, tert-butyl, tert-amyl, 2-ethylhexyl, n-octyl, undecyl, lauryl, heptadecyl, octadecyl, eicosyl and tricontyl; when said radicals, are alkenyl, they are, for example, allyl and oleyl; when said radicals are phenylalkyl, they are, for example, benzyl, phenethyl, α-methylbenzyl and α,α-dimethylbenzyl; when said radicals are aryl, they are, for example, phenyl, naphthyl, or when substituted by alkyl are, for example tolyl and xylyl; when said radicals are alkyl interrupted by -O-, they are, for example, 3-oxaamyl and 3,6-dioxaoctyl; when A is alkylene, said alkylene interrupted by -O-, A is, for example, ethylene, trimethylene, tetramethylene, hexamethylene, octamethylene, 3-oxapentamethylene and 3,6-dioxaoctamethylene; then A is arylene, A is, for example, o-phenylene, m-phenylene, p-phenylene and naphthylene; when A is alkanetriyl, it is, for example, 1,2,3-propanetriyl; and when A is alkanetetryl, A is, for example, 1,2,3,4-butanetetryl.

The substituted 1-oxy-4-acyloxypiperidines of this invention exhibit surprising process stabilization properties distinct from that shown by prior art compounds.

The instant compounds are structurally distinct from prior art compounds. Their, structures allow for the preparation of high molecular weight material of low volatility, enhanced compatibility with the substrate and low extractability. The instant compounds provide superior melt flow stabilization during polymer processing. The instant compounds also have superior hydrolytic stability over the state of the art phosphite stabilizers.

Substrates in which the compounds of this invention are particularly useful are polyolefins such as polyethylene and polypropylene; polystyrene, including especially impact polystyrene; ABS resin; elastomers such as e.g. butadiene rubber, EPM, EPDM, SBR and nitrile rubber; and lubricating oils. Especially preferred as substrates are polyethylene and polypropylene.

In general polymers which can be stabilized include
1. Polymers of monoolefins and diolefins, for example polypropylene, polyisobutylene, polybut-1-ene, polymethylpent-1-ene, polyisoprene or polybutadiene, as well as polymers of cycloolefins, for example of cyclopentene or norbornene, polyethylene (which may be crosslinked), for example high density polyethylene (HDPE), low density polyethylene (LDPE), linear low density polyethylene (LLDPE), branched low density polyethylene (BLDPE).
2. Mixtures of the polymers mentioned in 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefins and diolefins with each other or with other vinyl monomers, for example ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/but-1-ene, propylene/butadiene, isobutylene/isoprene, ethylene/alkyl acrylates, ethylene/alkyl methacrylates, ethylene/vinyl acetate copolymers and their copolymers with carbon monoxide or ethylene/acrylic acid copolymers and their salts (ionomers) and terpolymers of ethylene with propylene and a diene, such as hexadiene, dicyclopentadiene or ethylidene-norbomene; as well as mixtures of such copolymers and their mixtures with polymers mentioned in 1) above, for example polypropylene/ethylene-propylene-copolymers, LDPE/ethylene-vinyl acetate copolymers (EVA), LDPE/ethylene-acrylic acid copolymers (EAA), LLDPE/EVA, LLDPE/EAA and random or alternating polyalkylene/carbon monoxide-copolymers as well as mixtures thereof with other polymers, for example polyamides.
3a. Hydrocarbon resins (for example C₅-C₉) and hydrogenated modifications thereof (for example tackifiers) and mixtures of polyalkylenes and starch.
4. Polystyrene, poly(p-methylstyrene), poly(α-methylstyrene).
5. Copolymers of styrene or α-methylstyrene with dienes or acrylic derivatives, such as, for example, styrene/butadiene, styrene/acrylonitrile, styrene/alkyl methacrylate, styrene/maleic anhydride, styrene/butadiene/alkyl acrylate, styrene/butadiene/alkyl methacrylate styrene/acrylonitrile/methyl acrylate; mixtures of high impact strength from styrene copolymers and another polymer, for example from a polyacrylate, a diene polymer or an ethylene/propylene/diene terpolymer, and block copolymers of styrene, for example styrene/butadiene/styrene, styrene/isoprene/styrene, styrene/ethylene/butylene/styrene or styrene/ethylene/propylene/ styrene.
6. Graft copolymers of styrene or α-methylstyrene, for example styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed in 5), for example the copolymer mixtures known as ABS, MBS, ASA or AES polymers.
7. Halogen-containing polymers, such as polychloroprene, chlorinated rubbers, chlorinated or sulfochlorinated polyethylene, copolymers of ethylene and chlorinated ethylene, epichlorohydrin homo- and copolymers, polymers of halogenated vinyl compounds, for example polyvinyl chloride, polyvinylidene chloride, polyvinyl fluoride, polyvinylidene fluoride, as well as copolymers thereof, for example vinyl chloride/vinylidene chloride, vinyl chloride/vinyl acetate or vinylidene chloride/vinyl acetate copolymers.
8. Polymers derived from α,β-unsaturated acids and derivatives thereof, such as polyacrylates and polymethacrylates, polymethyl methacrylate impact-modified with butyl acrylate, polyacrylamides and polyacrylonitriles.
9. Copolymers of the monomers mentioned in 8) with each other or with other unsaturated monomers, for example acrylonitrile/ butadiene, acrylonitrile/alkyl acrylate, acrylonitrile/alkoxyalkyl acrylate or acrylonitrile/vinyl halide copolymers or acrylonitrile/alkyl methacrylate/butadiene terpolymers.
10. Polymers derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.
11. Homopolymers and copolymers of cyclic ethers, such as polyalkylene glycols, polyethylene oxide, polypropylene oxide or copolymers thereof with bisglycidyl ethers.
12. Polyacetals, such as polyoxymethylene and those polyoxymethylenes which contain ethylene oxide as comonomer; polyacetals modified with thermoplastic polyurethanes, acrylates or MBS.
13. Polyphenylene oxides and sulfides, and mixtures of pokyphenylene oxides with polystyrene or polyamides.
14. Polyurethanes derived from polyethers, polyesters or polybutadienes with terminal hydroxyl groups on the one hand an aliphatic or aromatic polyisocyantes on the other, as well as precursors thereof (polyisocyantes, polyols or prepolymers).
15. Polyamides and copolyamides derived from diamines and dicarboxylic acids and/or from aminocarboxylic acids or the corresponding lactams, such as polyamide 4, polyamide 6, polyamide 6/6, 6/10, 6/9, 6/12, 4/6, 12/12, polyamide 11, polyamide 12, aromatic polyamides obtained by condensation of m-xylene, diamine and adipic acid; polyamides prepared from hexamethylenediamine and isophthalic acid and/or terephthalic acid and optionally an elastomer as modifier, for example poly(2,4,4,-trimethylhexamethylene) terephthalamide or poly-m-phenylene isophthalamide. Further copolymers of the aforementioned polyamides with polyolefins, olefin copolymers, ionomers or chemically bonded or grafted elastomers; or with polyethers, as with polyethylene glycols, polypropylene glycols or polytetramethylene glycols. Polyamides or copolyamides modified with EPDM or ABS. Polyamides condensed during processing (RIM polyamide systems).
16. Polyureas, polyimides and polyamide-imides.
17. Polyesters derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly[2,2-(4-hydroxyphenyl)propane] terephthalate and polyhydroxybenzoates as well as block-copolyether-esters derived from polyethers having hydroxyl end groups.
18. Polycarbonates and polyester carbonates.
19. Polysulfones, polyether sulfones and polyether ketones.
20. Crosslinked polymers which are derived from aldehydes on the one hand and phenols, ureas and melamines on the other, such as pheno/formaldehyde resins, urea/formaldehyde resins and melamine/formaldehyde resins.
21. Drying and non-drying alkyd resins.
22. Unsaturated polyester resins derived from copolyesters of saturated and unsaturated dicarboxylic acids with polyhydric alcohols and vinyl compounds as crosslinking abents, and also halogenated modifications thereof of low inflammability.
23. Thermosetting acrylic resins derived from substituted acrylic esters, such as epoxy acrylates, urethane acrylates or polyester acrylates.
24. Alkyd resins, polyester resins or acrylate resins in admixture with melamine resins, urea resins, polyisocyanates or epoxy resins as crosslinking agents.
25. Crosslinked epoxy resins which are derived from polyepoxides, for example from bisglycidyl ethers or from cycloaliphatic diepoxides.
26. Natural polymers, such as cellulose, rubber, gelatin and chemically modified homologous derivatives thereof, such as cellulose acetates, cellulose propionates and cellulose butyrates, or the cellulose ethers, such as methyl cellulose; rosins and their derivatives.
27. Mixtures of polymers as mentioned above, for example PP/EPDM, polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.
29. Aqueous emulsions of natural or synthetic rubber, e.g. natural latex or latices of carboxylated styrene/butadiene copolymers.
30. Polysiloxanes such as the soft, hydrophilic polysiloxanes described, for example, in U.S. Patent No. 4,259,467; and the hard polyorganosiloxanes described, for example, in U.S. Patent No. 4,355,147.
31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.
32. Radiation curable compositions containing ethylenically unsaturated monomers or oligomers and a polyunsaturated aliphatic oligomer.
33. Epoxymelamine resins such as light-stable epoxy resins crosslinked by an epoxy functional coetherified high solids melamine resin such as LSE-4103 (Monsanto).

In general, the compounds of the present invention are employed in from about 0.01 to about 5% by weight of the stabilized composition, although this will vary with the particular substrate and application. An advantageous range is from about 0.05 to about 2%, and especially 0.1 to about 1%.

The stabilizers of the instant invention may readily be incorporated into the organic polymers by conventional techniques, at any convenient state prior to the manufacture of shaped articles therefrom. For example, the stabilizer may be mixed with the polymer in dry powder form, or a suspension or emulsion of the stabilizer may be mixed with a solution, suspension, or emulsion of the polymer. The resulting stabilized polymer compositions of the invention may optionally also contain an other stabilizer, examples of which are listed below, or mixtures thereof.

### 1. Antioxidants

1.1. Alkylated monophenols, for example 2,6-di-tert-butyl-4-methyl-phenol, 2-tert-butyl-4,6-dimethylphenol, 2,6-di-tert-butyl-4-ethylphenol, 2,6-di-tert-butyl-4-n-butylphenol, 2,6-di-tert-butyl-4-iso-butylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexyl-phenol, 2,6-di-tert-butyl-4-methoxymethylphenol, 2,6-di-nonyl-4-methylphenol, 2,4-dimethyl-6-(1′-methyl-undec-1′-yl)-phenol, 2,4-dimethyl-6-(1′-methylheptadec-1′-yl)-phenol, 2,4-dimethyl-6-(1′-methyl-tridec-1′-yl)-phenol and mixtures therof.
1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.
1.3. Hydroquinones and alkylated hydroquinones, for example 2,6-di-tert-butyl-4-methoxy-phenol, 2,5-di-tert-butylhydroquinone, 2,5-di-tert-amylhydroquinone, 2,6-diphenyl-4-octadecyloxyphenol, 2,6-di-tert-butyl-hydroquinone, 2,5-di-tert-butyl-4-hydroxyanisole, 3,5-di-tert-butyl-4-hydroxyanisole, 3,5-di-tert-butyl-4-hydroxyphenyl-stearate, bis-(3,5-di-tert-butyl-4-hydroxyphenyl)adipate.
1.4. Hydroxylated thiodiphenyl ethers, for example 2,2′-thiobis(6-tert-butyl-4-methylphenol), 2,2′-thiobis(4-octylphenol), 4,4′-thiobis(6-tert-butyl-3-methylphenol), 4,4′-thiobis(6-tert-butyl-2-methylphenol), 4,4′-thio-bis-(3,6-di-sec-amylphenol), 4,4′-bis-(2,6-dimethyl -4-hydroxyphenyl)-disulfide.
1.5. Alkylidenebisphenols, for example 2,2′-methylenebis(6-tert-butyl-4-methylphenol), 2,2′-methylenebis(6-tert-butyl-4-ethylphenol), 2,2′-methylenebis(4-methyl-6-(α-methylcyclohexyl)phenol], 2,2′-methylenebis(4-methyl-6-cyclohexylphenol), 2,2′-methylenebis(6-nonyl-4-methylphenol), 2,2′-methylenebis(4,6-di-tert-butylphenol), 2,2′-ethylidenebis(4,6-di-tert-butylphenol), 2,2′-ethylidenebis(6-tert-butyl-4-isobutylphenol), 2,2′-methylenebis[6-(α-methylbenzyl)-4-nonylphenol], 2,2′-methylenebis[6-(α,α-dimethylbenzyl)-4-nonylphenol], 4,4′-methylenebis(2,6-di-tert-butylphenol), 4,4′-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methyl-phenyl)-3-n-dodecylmercaptobutane, ethylene glycol bis[3,3-bis(3′-tert-butyl-4′-hydroxyphenyl)butyrate], bis(3-tert-butyl-4-hydroxy-5-methylphenyl)dicyclopentadiene, bis[2-(3′-tert-butyl-2′-hydroxy-5′-methylbenzyl)-6-tertbutyl-4-methylphenyl]terephthalate, 1,1-bis-(3,5-dimethyl-2-hydroxyphenyl)butan, 2,2-bis-(3,5-di-tert.-butyl-4-hydroxyphenyl)-propan, 2,2-bis-(5-tert.-butyl-4-hydroxy-2-methylphenyl)-4-n-dodecylmercapto-butan, 1,1,5,5-tetra-(5-tert.-butyl-4-hydroxy-2-methylphenyl)-pentan.
1.6. O-, N- and S-benzyl compounds, for example: 3,5,3′,5′-tetra-tert.-butyl-4,4′-dihydroxydibenzylether, octadecyl-4-hydroxy-3,5-dimethylbenzyl-mercaptoacetate, tris-(3,5-di-tert.-butyl-4-hydroxybenzyl)-amine, bis-(4-tert.-butyl-3-hydroxy-2,6-dimethylbenzyl)dithioterephthalate, bis-(3,5-di-tert.-butyl-4-hydroxybenzyl)-sulfide, isooctyl-3,5-di-tert.-butyl-4-hydroxybenzyl-mercaptoacetate.
1.7. Hydroxybenzylated Malonates, for example dioctadecyl-2,2-bis-(3,5-di-tert.-butyl-2-hydroxybenzyl)-malonate, di-octadecyl-2-(3-tert.-butyl-4-hydroxy-5-methylbenzyl)-malonate, di-dodecylmercaptoethyl-2,2-bis-(3,5-di-tert.-butyl-4-hydroxybenzyl)-malonate, Di-[4-(1,1,3,3-tetramethylbutyl)-phenyl]-2,2-bis-(3,5-di-tert.-butyl-4-hydroxybenzyl)-malonate.
1.8. Hydroxybenzyl-Aromatics, for example 1,3,5-tris-(3,5-di-tert.-butyl-4-hydroxybenzyl)-2,4,6-trimethylbenzene, 1,4-bis-(3,5-di-tert.-butyl-4-hydroxybenzyl)-2,3,5,6-tetramethylbenzene, 2,4,6-tris-(3,5-di-tert.-butyl-4-hydroxybenzyl)-phenol.
1.9. Triazine Compounds, for example 2,4-bis-octylmercapto-6-(3,5-di-tert.-butyl-4-hydroxyanilino)-1,3,5-triazine, 2-octylmercapto-4,6-bis-(3,5-di-tert.-butyl-4-hydroxyanilino)-1,3,5-triazine, 2-octylmercapto-4,6-bis-(3,5-di-tert.-butyl-4-hydroxyphenoxy)-1,3,5-triazine, 2,4,6-tris-(3,5-di-tert.-butyl-4-hydroxyphenoxy)-1,2,3-triazine, 1,3,5-tris-(3,5-ditert.-butyl-4-hydroxybenzyl)-isocyanurate, 1,3,5-tris-(4-tert.-butyl-3-hydroxy-2,6-di-methylbenzyl)-isocyanurate, 2,4,6-tris-(3,5-di-tert.-butyl-4-hydroxyphenylethyl)-1,3,5-triazine, 1,3,5-tris-(3,5-di-tert.butyl-4-hydroxyphenylpropionyl)-hexahydro-1,3,5-triazine, 1,3,5-tris-(3,5-dicyclohexyl-4-hydroxybenzyl)-isocyanurate.
1.10. Benzylphosphonates, for example dimethyl-2,5-di-tert.-butyl-4-hydroxybenzylphosphonate, diethyl-3,5-di-tert.-butyl-4-hydroxybenzylphosphonate, dioctadecyl-3,5-di-tert.-butyl-4-hydroxybenzylphosphonate, dioctadecyl-5-tert.-butyl-4-hydroxy-3-methylbenzylphosphonate, Ca-salt of the 3,5-di-tert.-butyl-4-hydroxybenzyl-phosphonic acid monoethylester.
1.11. Acylaminophenols, for example lauric acid 4-hydroxyanilide, stearic acid 4-hydroxyanilide, octyl N-(3,5-di-tert-butyl-4-hydroxyphenyl)-carbamate.
1.12. Esters of β-(3,5-di-tert-butyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
1.13. Esters of β-(5-tert-butyl-4-hydroxy-3-methylphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
1.14 Esters of β-(3,5-dicyclohexyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
1.15 Esters of 3,5-di-tert.-butyl-4-hydroxyphenyl acetic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N′-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
1.16. Amides of β-(3,5-di-tert-butyl-4-hydroxyphenyl)propionic acid e.g. N,N′-bis(3,5-di-tert-butyl-4-hydroxyphenylpropionyl)hexamethylene-diamine, N,N′-bis(3,5-di-tert-butyl-4-hydroxyphenylpropionyl)tri-methylene-diamine, N,N′-bis(3,5-di-tert-butyl-4-hydroxyphenylpropionyl)-hydrazine.

### 2. UV absorbers and light stabilisers

2.1. 2-(2′-Hydroxyphenyl)benzotriazoles, for example the 5′-methyl, 3′,5′-di-tert-butyl, 5′-tert-butyl, 5′-(1,1,3,3-tetramethylbutyl), 5-chloro-3′,5′-di-tert-butyl, 5-chloro-3′-tert-butyl-5′-methyl, 3′-sec-butyl-5′-tert-butyl, 4′-octoxy, 3′,5′-di-tert-amyl and 3′,5′-bis(α,α- dimethylbenzyl), mixture of 5-chloro-3′-tert.-butyl-5′-(2-octyloxycarbonylethyl)-and 5-chloro-3′-tert.-butyl-5′-[2-(2-ethylhexyloxy)-carbonylethyl]-, 5-chloro-3′-tert.-butyl-5′-(2-methoxycarbonylethyl)-, 3′-tert.-butyl-5′-(2-methoxycarbonylethyl)-, 3′-tert.-butyl-5′-(2-octyloxycarbonylethyl)-, 3′-tert.-butyl-5′-[2-(2-ethylhexyloxy)-carbonylethyl]-, 3′-dodecyl-5′-methyl- and 3′-tert.-butyl-5′-(2-isooctyloxycarbonylethyl)-2′-hydroxyphenyl-2H-benztriazole(2), 2,2′-methylene-bis[4-(1,1,3,3-tetramethylbutyl)-6-benztriazole-2-yl-phenol]; product of ester interchange of 2-[3'-tert.-butyl-5'-(2-methoxycarbonylethyl)-2'-hydroxy-phenyl]-2H-benztriazole with polyethylene glycol 300; [R-CH₂CH₂-COO(CH₂)₃⁆₂ with R=3'-tert.-butyl-4'-hydroxy-5'-2H-benzotriazole-2-yl-phenyl.
2.2. 2-Hydroxybenzophenones, for example the 4-hydroxy, 4-methoxy, 4-octoxy, 4-decyloxy, 4-dodecyloxy, 4-benzyloxy, 4,2',4'-trihydroxy and 2'-hydroxy-4,4'-dimethoxy derivatives.
2.3. Esters of substituted and unsubstituted benzoic acids, as for example 4-tert.butylphenyl salicylate, phenyl salicylate, octylphenyl salicylate, dibenzoylresorcinol, bis-(4-tert.butylbenzoyl)-resorcinol, benzoylresorcinol, 2,4-di-tert.butylphenyl 3,5-di-tert.butyl-4-hydroxybenzoate, hexadecyl 3,5-di-tert.butyl-4-hydroxybenzoate, octadecyl 3,5-di-tert.-butyl-4-hydroxybenzoate, 2 methyl-4,6-di-tert.-butylphenyl 3,5-di-tert.-butyl-4-hydroxybenzoate.
2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxy-cinnamate, butyl α-cyano-β-methyl-p-methoxy-cinnamate, methyl α-carbomethoxy-p-methoxycinnamate and N-(β-carbomethoxy-β-cyanovinyl)-2-methylindoline.
2.5. Nickel compounds, for example nickel complexes of 2,2'-thio-bis-[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of 4-hydroxy-3,5-di-tert-butylbenzyl-phosphonic acid monoalkyl esters, e.g. of the methyl or ethyl ester, nickel complexes of ketoximes, e.g. of 2-hydroxy-4-methyl-phenyl undecyl ketoxime, nickel complexes of 1-phenyl-4-lauroyl-5-hydroxypyrazole, with or without additional ligands.
2.6. Sterically hindered amines, for example bis(2,2,6,6-tetramethyl-piperidyl) sebacate, bis-(2,2,6,6-tetramethyl-piperidyl) succinate, bis(1,2,2,6,6-pentamethylpiperidyl) sebacate, bis(1,2,2,6,6-pentamethylpiperidyl) n-butyl-3,5-di-tert-butyl-4-hydroxy-benzylmalonate, the condensation product of 1-(2-hydroxyethyl)-2,2,6,6-tetramethyl-4-hydroxypiperidine and succinic acid, the condensation product of N,N'-bis(2,2,6,6-tetramethyl-4-piperidyl)-hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4-piperidyl)-1,2,3,4-butane-tetracarboxylate, 1,1′-(1,2-ethanediyl)bis-(3,3,5,5-tetramethylpiperazinone), 4-benzoyl-2,2,6,6-tetramethylpiperidine, 4-stearyloxy-2,2,6,6-tetramethylpiperidine, bis-(1,2,2,6,6-pentamethylpiperidyl)-2-n-butyl-2-(2-hydroxy-3,5-di-tert.-butylbenzyl) malonate, 3-n-octyl-7,7,9,9-tetramethyl-1,3,8-triazasprio[4.5]decan-2,4-dion, bis-(1-octyloxy-2,2,6,6-tetramethylpiperidyl) sebacate, bis-(1-octyloxy-2,2,6,6-tetramethylpiperidyl) succinate, product of condensation of N,N′-bis-(2,2,6,6-tetramethyl-4-piperidyl)-hexamethylene diamine and 4-morpholino-2,6-dichloro-1,3,5-triazine, product of condensation of-chloro-4,6-di-(4-n-butylamino-2,2,6,6-tetramethylpiperidyl)-1,3,5-triazine and 1,2-bis-(3-aminopropylamino)ethane, product of condensation of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]decane-2,4-dion, 3-dodecyl-1-(2,2,6,6-tetramethyl-4-piperidyl)pyrrolidin-2,5-dion, 3-dodecyl-1-(1,2,2,6,6-pentamethyl-4-piperidyl)-pyrrolidin-2,5-dion.
2.7. Oxalic acid diamides, for example 4,4′-dioctyloxyoxanilide, 2,2′-dioctyloxy-5,5′-di-tert-butyloxanilide, 2,2′-didodecyloxy-5,5′-di-tert-butyloxanilide, 2-ethoxy-2′-ethyloxynilide, N,N′-his(3-dimethylamino-propyl)oxalamide, 2-ethoxy-5-tertbutyl-2′-ethyloxanilide and its mixture with 2-ethoxy-2′-ethyl-5,4′-di-tert-butyloxanilide and mixtures of ortho- and para-methoxy-disubstituted oxanilides and mixtures of o- and p-ethoxy-disubstituted oxanilides.
2.8. 2-(2-hydroxyphenyl)-1,3,5-triazines, for example 2,4,6-tris(2-hydroxy-4-octyloxyphenyl)-1,3,5-triazine, 2-(2-hydroxy-4-octyloxy-phenyl)-4,6-bis(2,4-dimethylphenyl)-1,3,5-triazine, 2-(2,4-dihydroxy-phenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-[2-hydroxy-4-(2-hydroxy-3-butyloxy-propyloxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine.
3. Metal deactivators, for example N,N′-diphenyloxalic acid diamide, N-salicylal-N′-salicyloylhydrazine, N,N′-bis(salicyloyl)hydrazine, N,N′-bis(3,5-di-tert-butyl-4-hydroxyphenylpropionyl)hydrazine, 3-salicyl-oylamino-1,2,4-triazole, bis(benzylidene)-oxalodihydrazide, Oxanilide, isophtalic acid dihydrazide, sebacic acid-bis-phenyl-hydrazide, N,N′-diacetal-adipinic acid dihydrazide, N,N′-bis-salicyloyl-oxalic acid dihydrazide, N,N′-bis-salicyloyl-thiopropionic acid dihydrazide.
4. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diiso- decyl pentaerythritol diphosphite, bis(2,4-di-tert.-butylphenyl) pentaerythritol diphosphite, bis-(2,6-di-tert.-butyl-4-methylphenyl)-pentaeryt hritol diphosphite, bis-isodecyloxy-pentaerythritol diphosphite, bis-(2,4-di-tert.-butyl-6-methylphenyl)-pentaerythritol diphosphite, bis-(2,4,6-tri-tert.-butylphenyl)-pentaerythritol diphsophite, tristearyl sorbitol triphosphite, tetrakis(2,4-di-tert-butylphenyl) 4,4′-biphenylene diphosphonite, 6-isooctyloxy-2,4,8,10-tetra-tert.-butyl-12H-dibenz[d,g]-1,3,2-dioxaphosphocin, 6-fluoro-2,4,8,10-tetra-tert.-butyl-12-methyl-dibenz[d,g]-1,3,2-dioxaphosphocin.
5. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecyl-mercapto)propionate.
6. Polyamide stabilisers, for example, copper salts in combination with iodides and/or phosphorus compounds and salts of divalent manganese.
7. Basic co-stabilisers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg behenate, Mg stearate, Na ricinoleate and K palmitate, antimony pyrocatecholate or zinc pyrocatecholate.
8. Nucleating agents, for example, 4-tert.butyl-benzoic acid, adipic acid, diphenylacetic acid.
9. Fillers and reinforcing agents, for example, calcium carbonate, silicates, glass fibres, asbestos, talc, kaolin, mica, barium sulfate, metal oxides and hydroxydes, carbon black, graphite.
10. Other additives, for example, plasticisers, lubricants, emulsifiers, pigments, optical brighteners, flameproofing abents, antistatic abents and blowing agents.

Frequently the stabilized polymer compositions additionally contain from about 0.01 to about 5%, preferably from about 0.025 to about 2%, and especially from about 0.1 to about 1% by weight of various other stabilizers, such as the materials listed above.

Polymer compositions according to the invention preferably contain as other stabilizer a phenolic antioxidant and/or a hindered amine. Some examples for phenolic antioxidants are the compounds listed above under items 1.1., 1.2. and 1.5.. Some examples for hindered amines are the compounds listed above under item 2.6..

The phenolic antioxidant of particular interest is selected from the group consisting of n-octadecyl 3,5-di-tert-butyl-4-hydroxyhydrocinnamate, neopentanetetrayl tetrakis(3,5-di-tert-butyl-4-hydroxyhydrocinammate), di-n-octadecyl 3,5-di-tert-butyl-4-hydroxybenzylphosphonate, 1,3,5-tris(3,5-di-tert-butyl-4-hydroxybenzyl)isocyanurate, thiodiethylene bis(3,5-di-tert-butyl-4-hydroxyhydrocinnamate), 1,3,5-trimethyl-2,4,6-tris(3,5-di-tert-butyl-4-hydroxybenzyl)benzene, 3,6-dioxaoctamethylene bis)3-methyl-5-tert-butyl-4-hydroxyhydrocinnamate), 2,6-di-tert-butyl-p-cresol, 2,2′-ethylidene-bis(4,6-di-tert-butylphenol), 1,3,5-tris(2,6-dimethyl-4-tert-butyl-3-hydroxybenzyl)isocynurate, 1,1,3,-tris(2-methyl-4-hydroxy-5-tert-butylphenyl)butane, 1,3,5-tris[2-(3,5-di-tert-butyl-4-hydroxyhydrocinnamoyloxy)ethyl]isocyanurate, 3,5-di-(3,5-di-tert-butyl-4-hydroxybenzyl)mesitol, hexamethylene bis(3,5-di-tert-butyl-4-hydroxyhydrocinnamate), 1-(3,5-di-tert-butyl-4-hydroxyanilinol-3,5-di(octylthio)-s-triazine, N,N′-hexamethylene-bis(3,5-di-tert-butyl-4-hydroxyhydrocinnamamide), calcium bis(ethyl-3,5-di-tert-butyl-4-hydroxybenzylphosphonate), ethylene bis[3,3-di(3-tert-butyl-4-hydroxyphenyl)butyrate], octyl 3,5-di-tert-butyl-4-hydroxybenzylmercaptoacetate, bis(3,5-di-tert-butyl-4-hydroxyhydrocinnamoyl)hydrazide, and N,N′-bis[2-(3,5-di-tert-butyl-4-hydroxyhydrocinnamoyloxy)-ethyl]-oxamide.

A most preferred phenolic antioxidant is neopentanetetrayl tetrakis(3,5-di-tert-butyl-4-hydroxyhydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-di-tert-butyl-4-hydroxybenzyl)isocyanurate, 2,6-di-tert-butyl-p-cresol or 2,2′-ethylidene-bis-(4,6-di-tert-butylphenol).

The hindered amine compound of particular interest is selected from the group consisting of bis(2,2,6,6-tetramethylpiperidin-4-yl) sebacate, bis(1,2,2,6,6-pentamethylpiperidin-4-yl) sebacate, di(1,2,2,6,6-pentamethylpiperidin-4-yl)-(3,5-di-tert-butyl-4-hydroxybenzyl)butylmalonate, 4-benzoyl-2,2,6,6-tetramethylpiperidine, 4-stearyloxy-2,2,6,6-tetramethylpiperidine, 3-n-octyl-7,7,9,9-tetramethyl-1,3,8-triaza-spiro-[4.5]decane-2,4-dione, tris(2,2,6,6-tetramethylpiperidin-4-yl) nitrilotriacetate, 1,2-bis-(2,2,6,6-tetramethyl-3-oxopiperazin-4-yl)ethane, 2,2,4,4-tetramethyl-7-oxa-3,20-diaza-21-oxodispiro[5.1.11.2]heneicosane, polycondensation product of 2,4-dichloro-6-tert-octylamino-s-triazine and 4,4′-hexamethylenebis(amino-2,2,6,6-tetramethylpiperidine), polycondensation product of 1-(2-hydroxyethyl)-2,2,6,6-tetramethyl-4-hydroxypiperidine and succinic acid, polycondensation product of 4,4′-hexamethylenebis-(amino-2,2,6,6-tetramethylpiperidine) and 1,2-dibromoethane, tetrakis(2,2,6,6-tetramethylpiperidin-4-yl)-1,2,3,4-butanetetracarboxylate, tetrakis(1,2,2,6,6-pentamethylpiperidin-4-yl)1,2,3,4-butanetetracarboxylate, polycondensation product of 2,4-dichloro-6-morpholino-s-triazine and 4,4′-hexamethylenebis(amino-2,2,6,6-tetramethylpiperidine), N,N',N'',N'''-tetrakis[(4,6-bis(butyl-2,2,6,6-tetramethyl-piperidin-4-yl)-amino-s-triazin-2-yl]-1,10-diamino-4,7-diazadecane, mixed [2,2,6,6-tetramethylpiperidin-4-yl/β,β,β′,β′-tetramethyl-3,9-(2,4,8,10-tetraoxaspiro[5.5]-undecane)diethyl]1,2,3,4-butanetetracarboxylate, mixed [1,2,2,6,6-pentamethylpiperidin-4-yl/β,β,β′,β′-tetramethyl-3,9-(2,4,8,10-tetraoxaspiro[5.5]undecane)diethyl] 1,2,3,4-butanetetracarboxylate, octamethylene bis(2,2,6,6-tetramethylpiperidin-4-carboxylate), 4,4′-ethylenebis(2,2,6,6-tetramethylpiperazin-3-one) and bis(1-octyloxy-2,2,6,6-tetramethylpiperidin-4-yl) sebacate.

A most preferred hindered amine compound is bis(2,2,6,6-tetramethylpiperidin-4-yl) sebacate, the polycondensation product of 1-(2-hydroxyethyl)-2,2,6,6-tetramethyl-4-hydroxypiperdine and succinic acid, the polycondensation product of 2,4-dichloro-6-tert-octylamino-s-triazine and 4,4′-hexamethylenebis(amino-2,2,6,6-tetramethylpiperidine), N,N',N'',N'''-tetrakis[(4,6-bis(butyl-(2,2,6,6-tetramethyl-piperidin-4-yl)amino)-s-triazine-2-yl]-1,10-diamino-4,7-diazadecane or bis(1-octyloxy-2,2,6,6-tetramethylpiperidin-4-yl) sebacate.

The following examples are presented for the purpose of illustration only and are not to be construed to limit the nature or scope of the instant invention in any manner whatsoever. Percentages given are by weight if not otherwise indicated.

### Example 1

### 1-Allyl-4-hydroxypiperidine

Into a mixture of 4-hydroxypiperidine (75 g, 0.74 mol) and potassium carbonate (104 g, 0.75 mol) in ethanol (250 ml) is added allyl bromide (65 ml, 0.74 mol) in four equal portions over a three-hour period. After the addition is complete, the reaction mixture is stirred at ambient temperature until the disappearance of the reactants, as determined by TLC analysis (approximately six hours). The insoluble solids formed are then removed by filtration and the filtrate is concentrated in vacuo. The residue is purified by flash chromatography on silica gel with a mixture of 25% (vol.) methanol in ethyl acetate as the solvent to give 53.2 g (51% yield) of the title compound as a yellow oil.

¹³CNMR (90 MHz)(Benzene-d6); (ppm); 136.2; 117.3; 66.9; 61.2; 51.1; 34.8.

### Example 2

### 1-Allyloxy-4-hydroxypiperidine

Into a solution of 1-allyl-4-hydroxypiperidine, prepared in Example 1, (5 g, 0.35 mol) in dichloromethane (50 ml) at -5°C is added dropwise a solution of 85% active m-chloroperbenzoic acid (7.2 g, 0.35 mol) in dichloromethane (200 ml). After the addition is complete, the reaction mixture is warmed to ambient temperature with stirring. After two hours, the reaction mixture is concentrated in vacuo to give a red residue which is purified by flash chromatography on basic alumina with 40% (vol.) methanol in ethyl acetate as the solvent to give 2.5 g of a brown oil. The brown oil is dissolved into 10 ml of toluene and refluxed for 2.5 hours. The solution is concentrated in vacuo and resultant residue is purified by flash chromatography on silica gel with 25% (vol.) ethyl acetate in hexane as solvent to give 0.95 g (17% yield) of the title compound as a yellow oil.

¹HNMR (200 MHz)(Benzene-d6) (ppm); 1.5-20 (m, 5H); 2.72 (bs, 2H); 3.22 (m, 2H); 3.66 (m, 1H); 4.30 (d of t, 2H); 5.15 (d of d of t,1H); 5.30 (m, 1H); 6.07 (q of t, 1H).

### Example 3

### 1-(2-Methoxycarbonyl)ethylpiperidin-4-yl Stearate

A mixture of 4-hydroxypiperidine (35.5 g, 0.35 mol) and methyl acrylate (30.1 g, 0.35 mol) is heated to 74°C. After five hours, excess methyl acrylate (6.0 g, 0.07 mol) is added to the reaction mixture. After an additional half hour at 74°C, unreacted methyl acrylate is removed in vacuo to give 66.9 g of crude yellowish oil. A mixture of the yellowish crude oil (12.35 g), ether (100 ml) and triethylamine (6.7 g, 8.80 ml, 0.07 mol) is added dropwise to a solution of stearoyl chloride (20 g, 0.07 mol) in ether (150 ml) at -5°C. After the addition is complete, the reaction mixture is allowed to warm to ambient temperature with stirring. The reaction is considered complete upon disappearance of the reactants as determined by inspection of the IR spectrum of an aliquot wherein the carbonyl stretch band of the stearoyl chloride at 1800 cm⁻¹ and the hydroxyl stretch band of the 4-hydroxypiperidine derivative at 3500 cm⁻¹ are absent. The precipitate which has formed is removed by filtration. The filtrate is concentrated in vacuo to give 32 g of the title compound as a pale yellow solid: m.p. 46-50°C.

IR (CH₂Cl₂) 1740 cm⁻¹.

### Example 4

### 1-Hydroxypiperidin-4-yl Stearate

A mixture of 50% active m-chloroperbenzoic acid (9.8 g, 0.048 mol) and chloroform (130 ml) is added dropwise to a mixture of 1-(2-methoxycarbonyl)ethylpiperidin-4-yl stearate (22 g, 0.05 mol) and chloroform (130 ml) at 13°C under nitrogen. Reaction is complete in four hours. Removal of solvent gives a yellowish crude residue which is purified by flash chromatography on basic alumina with ethyl acetate as solvent to give 4.6 g (25% yield) of the title compound as a white solid: m.p. 75-80°C.

| Analysis: | | | |
|---|---|---|---|
| Calcd. for C₂₃H₄₅NO₃: | C, 72.0; | H, 11.8; | N, 3.6. |
| Found: | C, 71.7; | H, 11.7; | N, 3.5. |

### Example 5

### Bis[1-(2-methoxycarbonyl)ethylpiperidin-4-yl] Sebacate

The procedure of Example 3 is repeated using 18.7 g 0.1 mol) of 4-hydroxy-1-(2-methoxycarbonyl)ethylpiperidine, 10.1 mL (0.048 mol)of sebacoyl chloride, 14 mL (0.1 mol) of triethylamine and 240 mL of diethyl ether. The isolated reaction mixture is purified by flash chromatography (silica gel; 95:5 v/v ethyl acetate: methanol solvent) to give 14.9 g (58% yield) of the title compound as a white solid: mp 55-59°C.

| Analysis: | | | |
|---|---|---|---|
| Calcd. for C₂₈H₄₈N₂O₈: | C, 62.2; | H, 9.0; | N, 5.2. |
| Found: | C, 62.4; | H, 9.0; | N, 5.1. |

### Example 6

### Bis(1-Hydroxypiperidin-4-yl) Sebacate

The procedure of Example 4 is repeated using 14 g (26 mmol) of bis[1-(2-methoxycarbonyl)ethylpiperidin-4-yl] sebacate, 10.5 g (52 mmol) of 85% active meta-chloroperbenzoic acid and 200 ml of ethyl acetate. The isolated reaction mixture is purified by HPLC (silica gel; 95:5 v/v ethyl acetate:methanol) followed by recrystallisation from ethyl acetate to give 3.6 g (35% yield) of the title compound as a white solid: mp 133-135°C.

| Analysis: | | | |
|---|---|---|---|
| Calcd. for C₂₀H₃₆N₂O₆: | C, 60.0; | H, 9.1; | N, 7.0. |
| Found: | C, 59.6; | H, 8.9; | N, 7.0. |

### Example 7

### Bis(1-allyloxy-3,5-dimethylpiperidin-4-yl) Sebacate

The general procedure of Example 3 is repeated using 1-allyloxy-3,5-dimethyl-4-hydroxypiperidine, sebacoyl chloride and triethylamine in diethyl ether solvent to give the title compound.

### Example 8

### Bis(1-allyloxypiperidin-4-yl) Sebacate

Following the general procedure of Example 1 using bis(1-hydroxypiperidin-4-yl) sebacate, potassium carbonate and allyl bromide in ethanol solvent, the title compound is obtained.

### Example 9

### 1-Allyloxypiperidin-4-yl Stearate

Repeating the general procedure of Example 1 using 1-hydroxy-piperdin-4-yl stearate, potassium carbonate and allyl bromide in ethanol solvent yields the title compound.

### Example 10

### Bis(1-benzyloxypiperidin-4-yl) Sebacate

The general procedure of Example 3 is repeated using 1-benzyloxy-4-hydroxypiperidine, sebacoyl chloride and triethylamine in diethyl ether solvent to give the title compound.

### Example 11

### 1-Benzyloxypiperidin-4-yl Stearate

The general procedure of Example 1 is repeated using 1-hydroxypiperidin-4-yl stearate, poassium carbonate and benzyl bromide in ethanol solvent to give the title compound.

### Example 12

### 4-(N-n-butyl-N-stearoylamino)-1-hydroxypiperidine

The general procedure of Example 4 is followed using 4-(n-butyl-N-stearoylamino)-1-(2-methoxycarbonyl)ethylpiperidine and 50% active m-chloroperbenzoic acid in chloroform to give the title compound.

### Example 13

### Process Stabilization of Polypropylene at 525°F (274°C)

This example illustrates the process stabilizing effectiveness of the instant compounds in polypropylene.

The base formulation comprises unstabilized, old technology polypropylene (PROFAX 6501, Himont) containing 0.075% by weight of calcium stearate. The test additive is incorporated into the polypropylene by dry blending or, when the additive is a liquid, using a minimum amount of methylene chloride solvent. The solvent is then removed by evaporation under reduced pressure. The stabilized resin formulation is extruded at 90 rpm from a 1 inch (2.54 cm) diameter extruder at 525°F (274°C) with a residence time of 90 seconds.

After each of the first and fifth extrusions, the melt flow rate (in grams/10 minutes) is determined by ASTM method D1238 on the pellets obtained from the extruder. The results are given in the table below.

| Additive* | Concentration (% by weight) | Melt Flow after Extrusion | |
|---|---|---|---|
| | | 1 | 5 |
| None | - | 10.5 | 61.7 |
| Compnd. of Example 6 | 0.075 | 2.9 | 6.1 |

| | | | |
|---|---|---|---|
| *Example 6 compound is bis(1-hydroxypiperidin-4-yl) sebacate. | | | |

The result exemplify the ability of the instant compounds to provide superior melt flow stabilization to polypropylene during processing.

### Example 14

### Process Stabilization of Polypropylene at 525°F (274°C)

This example illustrates the process stabilizing efficacy of the instant compounds in polypropylene in formulations containing a phenolic antioxidant. The results using the procedure described in Example 13 on polypropylene formulations containing an instant compound and a phenolic antioxidant are given in the table below.

| Additive* | Concentration (% by weight) | Melt Flow after Extrusion | |
|---|---|---|---|
| | | 1 | 5 |
| AO A | 0.075 | 6.7 | 20.2 |
| AO A plus Example 6 | 0.075 | 3.0 | 5.3 |
| | 0.075 | | |

| | | | |
|---|---|---|---|
| *AO A is neopentanetetrayl tetrakis(3,5-di-tert-butyl-4-hydroxyhydrocinnamate). Example 6 compound is bis(1-hydroxypiperidin-4-yl) sebacate. | | | |

These results exemplify the ability of the substituted 1-oxy-4-acyloxypiperidines in combination with a phenolic antioxidant to provide superior melt flow stabilization to polypropylene during processing.

### Example 15

### Process Stabilization of Polypropylene at 536°F (280°C)

This example illustrates the process stabilizing effectiveness of the instant compounds in polypropylene.

The base formuation comprises unstabilized, new technology polypropylene (PROFAX 6501, Himont) containing 0.1% by weight of calcium stearate. The test additives are incorporated into the polyproyplene by dry blending or, when the additive is a liquid, using a minimum amount of methylene chloride solvent. The solvent is then removed by evaporation under reduced pressure. The stabilized resin formulation is extruded at 80 rpm from a 1 inch (2.54 cm) diameter extruder at 536°F (280°C) with a residence time of 45 seconds.

After each of the first and fifth extrusions, the melt flow rate (in grams/10 minutes) is determined by ASTM method D1238 on the pellets obtained from the extruder. The results are given in the table below.

| Additive* | Concentration (% by weight) | Melt Flow after Extrusion | |
|---|---|---|---|
| | | 1 | 5 |
| None | - | 22 | 82 |
| AO A | 0.1 | 18 | 26 |
| AO A plus Example 4 Compound | 0.1 | 6.9 | 9.7 |
| | 0.05 | | |

| | | | |
|---|---|---|---|
| *AO A is neopentanetetrayl tetrakis(3,5-di-tert-butyl-4-hydroxyhydrocinnamate). | | | |

These results exemplify the ability of the substituted 1-oxy-4-acyloxypiperidines in combination with a phenolic antioxidant to provide superior melt flow stabilization to polypropylene during processing.

## Claims

1. A compound of formula I 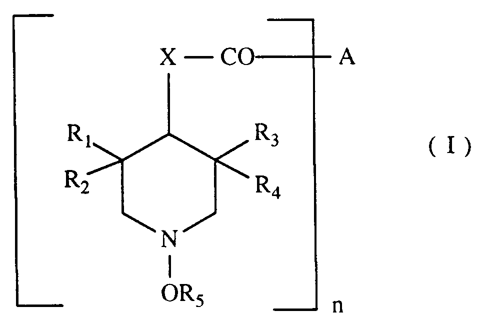 wherein
R₁, R₂, R₃ and R₄ are independently hydrogen; a linear or branched alkyl of 1 to 30 carbon atoms; said alkyl optionally terminated with -OR₆, -NR₇R₈, -SR₉, -COOR₁₀ or -CONR₁₁R₁₂, where R₆, R₇, R₈, R₉ and R₁₀ are independently alkyl of 1 to 20 carbon atoms or alkenyl of 3 to 18 carbon atoms, and R₁₁ and R₁₂ are independently hydrogen or the same meaning as R₆; or said alkyl interrupted by one or more -O-, -S-, -SO-, -SO₂-, -CO-, -COO-, -OCO-, -CONR₁₃-, -NR₁₃CO- or -NR₁₄- where R₁₃ and R₁₄ have the same meaning as R₁₁; alkenyl of 3 to 20 carbon atoms; aryl of 6 to 10 carbon atoms; or said aryl substituted by one to three substituents selected from the group consisting of alkyl of 1 to 20 carbon atoms, cycloalkyl of 5 to 12 carbon atoms and phenylalkyl of 7 to 15 carbon atoms;
R₅ is hydrogen, alkyl of 1 to 18 carbon atoms, cycloalkyl of 5 to 12 carbon atoms, allyl or benzyl;
X is -O- or -NE-,
E is hydrogen, alkyl of 1 to 18 carbon atoms or cycloalkyl of 5 to 12 carbon atoms,
A is a direct bond; an n-valent linear or branched aliphatic hydrocarbon radical containing 1 to 20 carbon atoms, or said radical interrupted by one or more -O-, -S-, -SO-, -SO₂-, -CO-, -COO-, -OCO-, -CONR₁₅-, -NR₁₅CO- or -NR₁₆- where R₁₅ and R₁₆ have the same meaning as R₁₁; an n-valent aromatic or aromatic- aliphatic hydrocarbon of 6 to 30 carbon atoms, or said radical interrupted by one or more -O-, -S-, -SO-,-SO₂-, -CO-, -COO-, -OCO-, -CONR₁₇, -NR₁₇CO- or -NR₁₈- where R₁₇ and R₁₈ have the same meaning as R₁₁; or A can be -O-, -S-, -NR₁₉- where R₁₉ has the same meaning as R₁₁; and
n is an integer of 1 to 4.

2. A compound according to claim 1 wherein R₁, R₂, R₃ and R₄ are independently hydrogen or methyl.

3. A compound according to claim 2 where R₁, R₂, R₃ and R₄ are each hydrogen.

4. A compound according to claim 1 where X is -O-.

5. A compound according to claim 1 wherein n is 1 or 2.

6. A compound according to claim 1 wherein A is alkyl of 1 to 18 carbon atoms when n is 1; and alkylene of 2 to 12 carbon atoms when n is 2.

7. A compound according to claim 5 wherein A is alkyl of 11 to 17 carbon atoms when n is 1; and alkylene of 2 to 8 carbon atoms when n is 2.

8. A compound according to claim 1 where R₅ is hydrogen, allyl or benzyl.

9. A compound according to claim 1 wherein R₁, R₂, R₃ and R₄ are independently hydrogen or methyl, n is 1 or 2, A is alkyl of 11 to 18 carbon atoms when n is 1, and alkylene of 2 to 12 carbon atoms when n is 2, and R₅ is hydrogen, allyl or benzyl.

10. A stabilized composition which comprises
(a) a synthetic polymer subject to thermal or oxidative degradation during processing, and
(b) an effective stabilizing amount of a compound according to claim 1.

11. A composition according to claim 10 wherein the synthetic polymer is a polyolefin.

12. A composition according to claim 11 wherein the polyolefin is polyethylene or polypropylene.

13. A composition according to claim 10 which additionally contains an other stabilizer.

14. A composition according to claim 13 wherein the other stabilizer is a phenolic antioxidant and/or a hindered amine.

15. A method for stabilizing a synthetic polymer against damage by themal or oxidative degradation during processing, wherein a compound of formula I according to claim 1 is added thereto as stabilizer.

16. Use of a compound of formula I according to claim 1 as stabilizer for synthetic polymers against damage by thermal or oxidative degradation during processing.

## Patentansprüche

1. Verbindung der Formel I 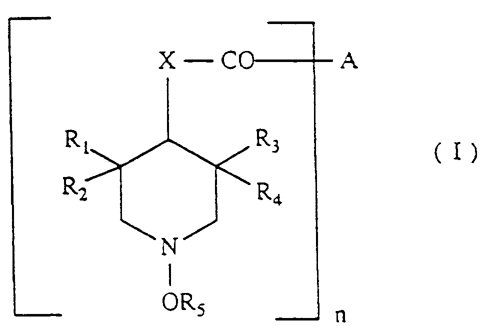 worin
R₁, R₂, R₃ und R₄ unabhängig Wasserstoff; lineares oder verzweigtes Alkyl mit 1 bis 30 Kohlenstoffatomen; dieses Alkyl, gegebenenfalls endständig versehen mit -OR₆, -NR₇R₈, -SR₉, -COOR₁₀ oder -CONR₁₁R₁₂, worin R₆, R₇, R₈, R₉ und R₁₀ unabhängig Alkyl mit 1 bis 20 Kohlenstoffatomen oder Alkenyl mit 3 bis 18 Kohlenstoffatomen bedeuten, und R₁₁ und R₁₂ unabhängig Wasserstoff bedeuten oder die Bedeutung von R₆ besitzen; oder dieses Alkyl, unterbrochen durch ein oder mehrere -O-, -S-, -SO-, -SO₂-, -CO-, -COO-, -OCO-, -CONR₁₃-, -NR₁₃CO- oder -NR₁₄-, worin R₁₃ und R₁₄ die Bedeutung von R₁₁ besitzen; Alkenyl mit 3 bis 20 Kohlenstoffatomen; Aryl mit 6 bis 10 Kohlenstoffatomen; oder dieses Aryl, substituiert durch einen bis drei Substituenten, ausgewählt unter Alkyl mit 1 bis 20 Kohlenstoffatomen, Cycloalkyl mit 5 bis 12 Kohlenstoffatomen und Phenylalkyl mit 7 bis 15 Kohlenstoffatomen, bedeuten;
R₅ Wasserstoff, Alkyl mit 1 bis 18 Kohlenstoffatomen, Cycloalkyl mit 5 bis 12 Kohlenstoffatomen, Allyl oder Benzyl bedeutet;
X für -O- oder -NE- steht,
E für Wasserstoff, Alkyl mit 1 bis 18 Kohlenstoffatomen oder Cycloalkyl mit 5 bis 12 Kohlenstoffatomen steht,
A eine direkte Bindung; ein n-wertiger linearer oder verzweigter, aliphatischer Kohlenwasserstoffrest mit 1 bis 20 Kohlenstoffatomen oder dieser Rest, unterbrochen durch ein oder mehrere -O-, -S-, -SO-, -SO₂-, -CO-, -COO-, -OCO-, -CONR₁₅-, -NR₁₅CO- oder -NR₁₆-, worin R₁₅ und R₁₆ die Bedeutung von R₁₁ besitzen; ein n-wertiger aromatischer oder aromatisch-aliphatischer Kohlenwasserstoffrest von 6 bis 30 Kohlenstoffatomen, oder dieser Rest, unterbrochen durch ein oder mehrere -O-, -S-, -SO-, -SO₂-, -CO-, -COO-, -OCO-, -CONR₁₇-, -NR₁₇CO- oder -NR₁₈-, worin R₁₇ und R₁₈ die Bedeutung von R₁₁ besitzen, ist; oder A für -O-, -S-, -NR₁₉-stehen kann, worin R₁₉ die Bedeutung von R₁₁ besitzt; und
n eine ganze Zahl von 1 bis 4 ist.

2. Verbindung gemäß Anspruch 1, worin R₁, R₂, R₃ und R₄ unabhängig Wasserstoff oder Methyl bedeuten.

3. Verbindung gemäß Anspruch 2, worin R₁, R₂, R₃ und R₄ jeweils Wasserstoff sind.

4. Verbindung gemäß Anspruch 1, worin X für -O- steht.

5. Verbindung gemäß Anspruch 1, worin n für 1 oder 2 steht.

6. Verbindung gemäß Anspruch 1, worin A Alkyl mit 1 bis 18 Kohlenstoffatomen ist, wenn n für 1 steht; und Alkylen mit 2 bis 12 Kohlenstoffatomen, wenn n für 2 steht.

7. Verbindung gemäß Anspruch 5, worin A Alkyl mit 11 bis 17 Kohlenstoffatomen ist, wenn n für 1 steht; und Alkylen mit 2 bis 8 Kohlenstoffatomen, wenn n für 2 steht.

8. Verbindung gemäß Anspruch 1, worin R₅ für Wasserstoff, Allyl oder Benzyl steht.

9. Verbindung gemäß Anspruch 1, worin R₁, R₂, R₃ und R₄ unabhängig Wasserstoff oder Methyl bedeuten, n für 1 oder 2 steht, A Alkyl mit 11 bis 18 Kohlenstoffatomen bedeutet, wenn n für 1 steht, und Alkylen mit 2 bis 12 Kohlenstoffatomen, wenn n für 2 steht, und R₅ Wasserstoff, Allyl oder Benzyl bedeutet.

10. Stabilisierte Zusammensetzung, umfassend
(a) ein synthetisches Polymeres, das einem thermischen oder oxidativen Abbau während der Verarbeitung unterliegt, und
(b) eine wirksame, stabilisierende Menge einer Verbindung gemäß Anspruch 1.

11. Zusammensetzung gemäß Anspruch 10, worin das synthetische Polymere ein Polyolefin ist.

12. Zusammensetzung gemäß Anspruch 11, worin das Polyolefin Polyethylen oder Polypropylen ist.

13. Zusammensetzung gemäß Anspruch 10, die zusätzlich einen weiteren Stabilisator enthält.

14. Zusammensetzung gemäß Anspruch 13, worin der weitere Stabilisator ein phenolisches Antioxidans und/oder ein gehindertes Amin ist.

15. Verfahren zur Stabilisierung eines synthetischen Polymeren gegenüber einer Schädigung durch thermischen oder oxidativen Abbau während der Verarbeitung, worin eine Verbindung der Formel I gemäß Anspruch 1 als Stabilisator zugesetzt wird.

16. Verwendung einer Verbindung der Formel I gemäß Anspruch 1 als Stabilisator für synthetische Polymere gegenüber einer Schädigung durch thermischen oder oxidativen Abbau während der Verarbeitung.

## Revendications

1. Composé de formule I 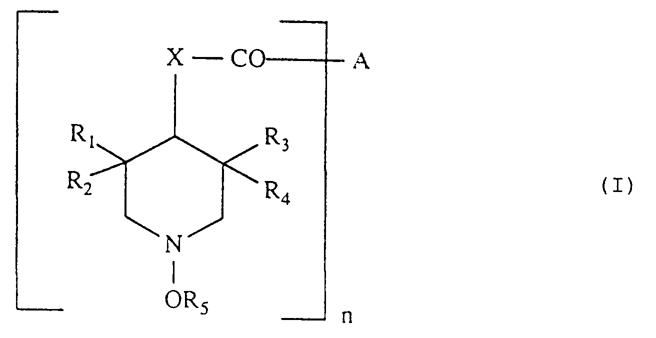 dans laquelle
R₁, R₂, R₃ et R₄, indépendamment les uns des autres, sont un atome d'hydrogène ; un alkyle linéaire ou ramifié avec 1 à 30 atomes de carbone ; ledit alkyle étant éventuellement terminé par des groupes -OR₆, -NR₇R₈, -SR₉, -COOR₁₀ ou -CONR₁₁R₁₂, où R₆, R₇, R₈, R₉ et R₁₀, indépendamment les uns des autres, représentent alkyle avec 1 à 20 atomes de carbone ou alcényle avec 3 à 18 atomes de carbone et R₁₁ et R₁₂, indépendamment les uns des autres, sont un atome d'hydrogène ou ont la même signification que R₆ ; ou ledit alkyle est interrompu par un ou plusieurs groupes -O-, -S-, -SO-, -SO₂-, -CO-, -COO-, -OCO-, -CONR₁₃-, -NR₁₃CO- ou -NR₁₄-, où R₁₃ et R₁₄ ont la même signification que R₁₁ ; alcényle avec 3 à 20 atomes de carbone ; aryle avec 6 à 10 atomes de carbone ; ou ledit aryle est substitué par un à trois substituants pris dans le groupe comportant alkyle avec 1 à 20 atomes de carbone, cycloalkyle avec 5 à 12 atomes de carbone et phénylalkyle avec 7 à 15 atomes de carbone ;
R₅ est l'hydrogène, alkyle avec 1 à 18 atomes de carbone, cycloalkyle avec 5 à 12 atomes de carbone, allyle ou benzyle ;
X représente -O- ou -NE-,
E est l'hydrogène, alkyle avec 1 à 18 atomes de carbone, ou cycloalkyle avec 5 à 12 atomes de carbone,
A est une liaison directe ; un radical hydrocarboné aliphatique linéaire ou ramifié n-valent comportant 1 à 20 atomes de carbone, ou ledit radical étant interrompu par un ou plusieurs groupes -O-, -S-, -SO-, -SO₂-, -CO-, -COO-, -OCO-, -CONR₁₅-, -NR₁₅CO- ou -NR₁₆-, où R₁₅ et R₁₆ ont la même signification que R₁₁ ; un radical hydrocarboné aromatique ou aromatique-aliphatique n-valent avec 6 à 30 atomes de carbone, ou ledit radical est interrompu par un ou plusieurs groupes -O-, -S-, -SO-, -SO₂-, -CO-, -COO-, -OCO-, -CONR₁₇-, -NR₁₇CO- ou -NR₁₈-, où R₁₇ et R₁₈ ont la même signification que R₁₁ ; ou A peut être -O-, -S-, -NR₁₉-, où R₁₉ a la même signification que R₁₁ ; et
n est un nombre entier de 1 à 4.

2. Composé selon la revendication 1, où R₁, R₂, R₃ et R₄, indépendamment les uns des autres, sont l'hydrogène ou méthyle.

3. Composé selon la revendication 2, où R₁, R₂, R₃ et R₄ sont chacun l'hydrogène.

4. Composé selon la revendication 1, où X est -O-.

5. Composé selon la revendication 1, où n vaut 1 ou 2.

6. Composé selon la revendication 1, où A est alkyle avec 1 à 18 atomes de carbone lorsque n est égal à 1 ; et alkylène avec 2 à 12 atomes de carbone lorsque n est égal à 2.

7. Composé selon la revendication 5, où A est alkyle avec 11 à 17 atomes de carbone lorsque n est égal à 1 ; et alkylène avec 2 à 8 atomes de carbone lorsque n est égal à 2.

8. Composé selon la revendication 1, où R₅ est l'hydrogène, allyle ou benzyle.

9. Composé selon la revendication 1, où R₁, R₂, R₃ et R₄, indépendamment les uns des autres, sont l'hydrogène ou méthyle, n vaut 1 ou 2, A est alkyle avec 11 à 18 atomes de carbone lorsque n vaut 1, et alkylène avec 2 à 12 atomes de carbone lorsque n vaut 2, et R₅ est l'hydrogène, allyle ou benzyle.

10. Composition stabilisée comprenant
(a) un polymère synthétique susceptible de dégradation par la chaleur ou par oxydation au cours de la mise en oeuvre, et
(b) une quantité efficace stabilisante d'un composé selon la revendication 1.

11. Composition selon la revendication 10, où le polymère synthétique est une polyoléfine.

12. Composition selon la revendication 11, où la polyoléfine est le polyéthylène ou le polypropylène.

13. Composition selon la revendication 10 qui comprend de plus un autre stabilisant.

14. Composition selon la revendication 13 où l'autre stabilisant est un antioxydant phénolique et/ou une amine à encombrement stérique.

15. Méthode de stabilisation d'un polymère synthétique contre la dégradation induite par la chaleur ou par l'oxygène au cours de la mise en oeuvre, où on ajoute à celle-ci un composé de formule I selon la revendication 1 en tant que stabilisant.

16. Utilisation d'un composé de formule I selon la revendication 1 comme stabilisant de polymères synthétiques contre la dégradation induite par la chaleur ou par la lumière au cours de la mise en oeuvre.
